## Europäisches Patentamt
## European Patent Office
## Office européen des brevets

(19)

(11) Veröffentlichungsnummer: **0 002 095**
**B1**

(12) **EUROPÄISCHE PATENTSCHRIFT**

(45) Veröffentlichungstag der Patentschrift: **26.08.81**

(21) Anmeldenummer: **78200310.7**

(22) Anmeldetag: **20.11.78**

(51) Int. Cl.³: **C 07 D 493/04, B 01 D 7/02**
**//(C07D493/04, 307/00)**

(54) **Verfahren zur fraktionierten Desublimation von Pyromellithsäuredianhydrid.**

(30) Priorität: **22.11.77 DE 2751979**

(43) Veröffentlichungstag der Anmeldung:
**30.05.79 Patentblatt 79/11**

(45) Bekanntmachung des Hinweises auf die Patenterteilung:
**26.08.81 Patentblatt 81/34**

(84) Benannte Vertragsstaaten:
**DE FR GB**

(56) Entgegenhaltungen:
**DE - A - 1 668 161**
**FR - A - 1 207 926**
**FR - A - 2 254 538**
**NL - A - 240 455**

(73) Patentinhaber: **CHEMISCHE WERKE HÜLS AG**
**Postfach 1320**
**D-4370 Marl 1 (DE)**

(72) Erfinder: **Haferkorn, Herbert**
**Horster Strasse 528**
**D-4250 Bottrop (DE)**

(74) Vertreter: **Steil, Hanna, Dipl.-Chem. et al,**
**RSP-PATENTE-PB 40 Postfach 2840**
**Holsterhauser Strasse 160**
**D-4690 Herne 2 (DE)**

# Verfahren zur fraktionierten Desublimation von Pyromellitsäuredianhydrid

## Beschreibung und Beispiele

Die wirtschaftliche Gewinnung von besonders reinen, sublimierbaren, oranischen Stoffen, d.h. solchen Stoffen, die unter den Arbeitsbedingungen unmittelbar aus dem dampfförmigen in den festen Zustand übergehen, aus einem heißen Gasgemisch, welches vornehmlich einem Reaktor zur Herstellung besagter Stoffe entnommen wird, und das als Verunreinigung Nebenprodukte enthält, die unter den erforderlichen Arbeitsbedingungen dampfförmig im Abgas verbleiben, hat in den letzten Jahren an Bedeutung gewonnen.

Die Abscheidung der sublimierbaren Stoffe aus diese enthaltenden Gasgemischen ist bekannt. Allen bekannten Verfahren gemeinsam ist das Erfordernis, die heißen Gase an gekühlten Flächen so weit absukühlen, daß der gewünschte Abscheidungsgrad für die zu gewinnende Substanz erreicht wird. Hierbei belegen sich die Wärmeaustauscherflächen meist mit Produkt, so daß eine periodische Entfernung erforderlich ist. Durch Schaber oder Bürsten lassen sich großräumige Sublimationskammern leicht entleeren. Doch ist infolge der relativ geringen Kammeroberflächen der Durchsatz bzw. die Raum-Zeit-Ausbeute gering. Die z.Z. wirtschaftlichsten Abscheider sind Rippenrohrkondensatoren, die sich jedoch mechanisch nicht entleeren lassen. Die Gewinnung des Sublimats bzw. Kondensates erfolgt hier durch periodisches Aufheizen der Rippenrohre auf Temperaturen oberhalb des Erstarrungspunktes der gewonnen Produkte und Ableitung der Produkte in Sammelbehälter. Nachteilig bei diesem Verfahren ist die Erfordernis von zwei wechselweise zu betreibenden Kondensatoren, von denen der eine beladen, während der andere abgeschmolzen wird. Weiterhin ist ein solcher Kondensator unbrauchbar für Produkte, die einem sehr hohen Schmelzpunkt aufweisen und/oder sich bei ihrem Schmelzpunkt zersetzen.

Zu den Produkten, die sich in Rippenrohrkondensatoren der üblichen Bauart nicht gewinnen lassen, gehört u.a. Pyromellitsäuredianhydrid (PMDA). Einmal liegt der Schmelzpunkt mit 285°C so hoch, daß ein Abschmelzen technische Schwierigkeiten bereiten würde, zum anderen tritt beim Erhitzen des PMDAs auf Schmelztemperatur eine Verfärbung des Produktes ein, die sich nach bisher bekannten Methoden nicht beseitigen läßt, es sei denn durch Destillation[1], die wegen des hohen Schmelzpunktes des Produktes ebenfalls nicht unerhebliche technische Probleme aufwirt.

Für die Abscheidung von PMDA aus den Reaktionsgasen sind in der Literatur verschiedene Verfahren bekannt geworden:

So wurde die Verwendung von Sublimationskammern mehrfach vorge-schlagen[2], wobei zur Verbesserung der Wirksamkeit dieser Kühlflächen und Prallbleche eingebaut werden können, oder eine Abkühlung der Gase durch Einspritzen von Wasser bewerkstelligt wird, wobei die Abkühlung jedoch 555°C/sec.[3] nicht überschreiten darf. Nachteilig bei letzterem Verfahren ist die Erhöhung des Taupunktes für Wasser, was eine vermehrte Bildung von Pyromellitsäure (PMS) zur Folge hat. Günstiger erscheint die Vermischung der heißen Reaktionsgase mit kalter Luft.[4] Hierbei entstehen aber sehr feine Kristalle, die infolge der Erhöhung der Strömungsgeschwindigkeit in verstärktem Maße aus den Sublimationskammern ausgetragen werden. Zur Vermeidung derartiger Verluste sind Filter oder Wasserwäschen vorgeschlagen worden[5].

Wesentlich einfacher gestaltet sich die Wasserwäsche der gesamten Reaktionsgase. Dabei fällt das gesamte Produkt als PMS an[6], das dann nach verschiedenen Methoden wieder zum Dianhydrid dehydratisiert werden muß[7].

Nach einem anderen Verfahren werden die heißen PMDA-enthaltenden Reaktionsgase mit pneumatisch geförderten Feststoffkugeln in Berührung gebracht, die ihrerseits die Wärme aufnehmen und sich dabei mit dem sublimierenden Gut überziehen[8]. In einer separaten Anlage werden die Feststoffkugeln mechanisch (Prallwirkung) gereinigt und gekühlt, ehe sie wieder in die Sublimationskammer zurückgefördert werden. Dieses Verfahren erfordert verständlicherweise eine komplizierte Apparatur.

Es ist weiterhin bekannt, daß das bei der Gasphasenoxidation von tetraalkylierten Benzolen (z.B. Durol) anfallende PMDA mit einer Reihe von Nebenprodukten verunreinigt ist, deren Dampfdrücke höher sind als der des PMDA und/oder aufgrund ihrer größeren Verdünnung im Reaktionsgas einen tieferen Taupunkt besitzen. Hierzu gehören u.a. Trimellitsäureanhydrid, Dimethyl-, Monomethylphthalsäureanhydrid und Phthalsäureanhydrid als farblose Verunreinigung neben einer Reihe weiterer dunkel gefärbter Verbindungen. Zur Entfernung dieser Verunreinigungen ist eine Raffination erforderlich. Die vorgeschlagenen Verfahren haben meist eine Behandlung des Rohproduktes mit Lösungsmitteln, vornehmlich Ketonen oder Lösungsmittelgemischen[9], zum

[1] = DE—AS 23 07 570

[2] = DE—AS 20 09 086

[3] = DE—AS 16 18 786

[4] = FR—PS 20 82 822

[5] = DE—AS 15 93 232

[6] = DE—AS 21 51 238

[7] = DE—AS 21 51 239

[8] = DE—AS 22 43 689

[9] = DE—AS 16 43 061

Gegenstand, während ein weiteres Verfahren bekannt wurde, wonach heißes Inertgas oder Luft bei Tempereaturen zwischen 100 und 200°C in einer Menge von 1 bis 150 kg Gas/kg Roh-PMDA über oder durch das Rohprodukt geleitet wird, bis ein Reinheitsgrad von 99% erreicht ist[10].

Es ist auch bekannt, daß man reines PMDA erhalten kann, wenn die Desublimation des Rohproduktes bei Temperaturen zwischen 130 und 200°C durchgeführt wird.

In einem neueren Verfahren werden PMDA-haltige Reaktionsgase, die auf um 5—10°C oberhalb des Taupunktes für PMDA liegende Temperatur vorgekühlt wurden, senkrecht durch eine thermostatisierte, perforierte Kühlfläche geleitet, die es ermöglicht, die Gase auf eine Temperatur oberhalb des Taupunktes der Nebenprodukte abzukühlen, wobei an der Zuströmseite der Kühlfläche reines PMDA anfällt, das sich leicht mechanisch abtrennen läßt[11]. Apparaturen dieser Art lassen sich jedoch nur unter erheblichem Kostenaufwand für eine großtechnische PMDA-Gewinnung erstellen.

Es wurde nun festgestellt, daß beim Durchleiten von auf 5—10°C oberhalb des Taupunktes für PMDA vorgekühlten PMDA-haltigen Reaktionsgasen durch thermostatisierte Rohre, wobei eine Abkühlung der Gase auf eine Temperatur oberhalb des Taupunktes der anwesenden Verunreinigungen erreicht wird, das desublimierende PMDA zu einem Teil an der Rohrwandung anbackt, während ein anderer Teil als Kristall mit dem gekühlten Gasstrom das Rohr verläßt und nach bekannten Methoden (Zykon, Filter) abgetrennt werden kann.

Überraschenderweise wurde gefunden, daß das Verhältnis der anbackenden Kristalle zu den ausgetragenen Kristallen wächst bei der Zunahme der Strömungsgeschwindigkeit der Gase durch das Rohr. Das heißt, daß bei kleinen Strömungsgeschwindigkeiten ein wesentlicher Teil der desublimierenden PMDA-Kristalle im nachgeschalteten Sammelraum gewonnen und als freifließendes kristallines Produkt hoher Reinheit abgezogen werden kann.

Gegenstand der Erfindung ist daher ein Verfahren zur fraktionierten Desublimation von hochreinem Pyromellitsäuredianhydrid aus dieses enthaltenden Reaktionsgasen in Form freifließender Kristalle, wobei die in bekannter Weise vorgekühlten Reaktionsgase zur weiteren an sich bekannten Abkühlung auf Temperaturen oberhalb des Taupunktes der Nebenprodukte gebracht werden, dadurch gekennzeichnet, daß die Reaktionsgase mit niedrigen Strömungsgeschwindigkeiten von maximal 3 m/sec., insbesondere maximal 1 m/sec., parallel zu glattflächigen, thermostatisierten Kühlflächen geführt werden.

---

[10] = DE—AS 14 68 851

[11] = DE—AS 23 62 659

Die im Rohr festhaftenden Kristalle müssen periodisch nach bekannten Methoden entfernt werden (Waschen mit Wasser, Lauge oder Lösungsmittel, mechanische Räumung oder Abschmelzen). Unter niedriger Strömungsgeschwindigkeit ist eine solche zu verstehen, bei der die Gase das Rohr möglichst laminar durchströmen. Der fest anhaftende Teil der anfallenden PMDA-Kristalle im Rohr kann so unter 20% des Gesamtanfalles gesenkt werden.

Die Tatsache, daß gerade kleine Strömungsgeschwindigkeiten das Abscheidungsverhältnis im oben angegebenen Sinne beeinflussen, ist unerwartet. Vielmehr würde man die Verhinderung von Anbackungen bei hohen Gasgeschwindigkeiten vermuten, da hierbei eine mechanische Reibwirkung an der Wand auftritt.

Zusätzlich zur Tatsache des gereingen Anteils festgebackener Kristallabsetzungen im Rohr—verglichen mit den lose anfallenden Kristallen—führt die Fahrweise mit geringer Strömungsgeschwindigkeit zur Ausbildung größerer Kristalle, die sich in den nachgeschalteten Apparaturen (Zyklon, Filter) leichter abscheiden lassen.

Ähnliche Beibachtungen wurden auch gemacht, wenn—wie oben erwähnt—vorgekühlte PMDA-haltige Reaktionsgase an andersgearteten thermostatisierten Kühlflächen (z.B. Plattenkühler) entlanggeführt werden. Beim laminaren Strömen der Gase parallel zur Kühlfläche fällt—sofern die Abkühlung der Gase gewährleistet ist—reines PMDA in gut ausgeprägten Kristallen an, die sich leicht abscheiden lassen, während Absetzungen auf den Kühlplatten über einen längeren Zeitraum in nur untergeordneter Menge auftrenen. Bei hohen Gasgeschwindigkeiten sind auch an solchen Kühlflächen verstärkt Absetzungen zu beobachten.

Einige Beispiele sollen das erfindungsgemäße Verfahren deutlich machen.

Beispiel 1.

Aus einem Reaktionsofen zur katalytischen Oxidation von Isopropylpseudocumol (IPPC) steht ein Reaktionsgas an, das bei 430°C 4,7 g PMDA neben 0,9—1,2 g Verunreinigungen pro Nm[3] enthält. Der Taupunkt für PMDA liegt in diesem Gas bei etwa 200°C. Für die Erprobung von Desublimatoren wird diesem Gas ein Teilstrom entnommen und auf 210—220°C vorgekühlt (bei dieser Temperatur tritt noch keinerlei Desublimation auf).

37 Nm[3]/Std. dieses Reaktionsgases werden durch ein senkrecht angeordnetes 6 m langes Doppelmantelrohr aud V4A-Stahl mit 46 mm lichter Weite, das mit einem Ölkreislauf auf 130°C thermostatisiert wird, von oben nach unten geleitet, Es tritt, auf etwa 150°C abgekühlt, in einen unterhalb des Rohres angebrachten Abscheiderraum mit nachgeschaltetem Filter ein. Die mittlere Strömungsgeschwindigkeit im Rohr beträgt etwa 10 m/sec. Nach 47 1/2 Stunden sind 1757 Nm³ Reaktionsgas durchgesetzt. Es tritt ein merklicher Druckverlust im Rohr ein. Aus dem Sammelraum und dem Filter werden 2,5 kg PMDA hoher Reinheit (99,8%) ausgetragen. Im Desublimationsrohr verbleiben 5,0 kg PMDA, das eine harte, dichtgepackte Schale bildet, die der Rohrwandung fest anhaftet. Die im Desublimationsrohr verbliebene Menge beträgt 67% der Gesamtmenge, die durch Desublimation aus dem Reaktionsgas gewonnen werden kann. Sie wird durch Auswaschen mit heißen Wasser entfernt.

### Beispiel 2.

Durch die Apparatur des Beispiels 1 werden 9,7 Nm³/Std. des gleichen Reaktionsgases geleitet. Die mittlere Strömungsgeschwindigkeit im Rohr beträgt 2,7 m/sec. Nach 232 Stunden sind 2250 Nm³ durchgesetzt. Es tritt ein ähnlicher Druckverlust wie in Beispiel 1 auf. Aus dem Sammelraum und dem Filter werden 5,9 kg PMDA (99,8% Reinheit) gewonnen. 3,8 kg Produkt verbleiben als Schale im Desublimationsrohr. Im Vergleich zu den Schalen des Beispiels 1 ist nunmehr eine etwas geringere Festigkeit zu beobachten.

Im Desublimationsrohr verbleiben 39% des Gesamtproduktes.

### Beispiel 3.

Durch die Appatatur des Beispiels 1 werden 3,6 Nm³/Std. des Reaktionsgases geleitet. Die mittlere Strömungsgeschwindigkeit beträgt etwa 1 m/sec. In 410 Stunden sind 1475 Nm³ Reaktionsgas durchgesetzt. Eine Steigerung des Druckabfalls im Rohr ist nicht zu beibachten. Aus Sammelraum und Filter werden 4,7 kg PMDA (Reinheit 99,9%) gewonnen. Im Desublimationsrohr verbleiben 1,6 kg, die sich als eine sehr lockere Schale an der Rohrwandung absetzen. Sie besteht aus verfilzten groben Kristallen, die sich leicht von der Wand lösen lassen.

In Desublimationsrohr verbleiben 25% des Gesamtproduktes.

### Beispiel 4.

Anstelle des Doppelmantelrohres mit 46 mm lichter Weite (Beispiel 1—3) wird ein Doppelmantelrohr aus V4A-Stahl mit 100 mm lichter Weite verwendet. Beschickt wird dieses Rohr mit 16,1 Nm³/Std. der oben angegebenen Reaktionsgase. Die Temperaturen werden den Vorversuchen angeglichen. Die mittlere Strömungsgeschwindigkeit errechnet sich zu 1 m/sec. Über 487 Stunden werden 7832 Nm³ durchgesetzt. Ein Druckgefälle im Rohr tritt nicht auf. Es hat sich während der gesamten Laufzeit eine etwa 15 mm dicke Schicht an der Rohrwandung locker aufgebaut, die während des Abkühlens nach Versuchsende zum Teil abfällt. Aus Sammelraum und Filter werden 30,3 kg PMDA (Reinheit 99,8%) gewonnen. Weitere 3,1 kg (= 9,3% der Gesamtmenge) werden aus der Apparatur durch Wasserwäsche entfernt.

### Beispiel 5.

Durch einen Plattenkühler mit 7,5 m² wirksamer Kühlfläche, Plattenabstand 120 mm, Plattenlänge 1000 mm, wird ein Reaktionsstrom von 66 Nm³/Std. senkrecht von unten nach oben geleitet. Bei einem freien Querschnitt des Kühlers von 0.4 m² und einer mittleren Gastemperatur von 175°C (Eintrittstemperatur 200°C; Austrittstemperatur 150°C, bei einer Temperatur an der Kühlplatte von 130°C) errechnet sich eine mittlere Strömungsgeschwindigkeit von 7,5 cm/sec.

Nach 562 Stunden Betriebszeit wird der Versuch abgebrochen. Es sind 149,8 kg PMDA (Reinheit 99,8%) angefallen, die sich leicht aus dem Sammelraum räumen lassen. Auf den Kühlplatten befindet sich ein loser Belag von etwa 3—4 mm, der abgewaschen wird. Dabei handelt es sich um 6,75 kg PMDA (Reinheit 99,6%), entsprechend 4,3% des Gesamtanfalls.

### Patentansprüche

1. Verfahren zur fraktionierten Desublimation von hochreinem Pyromellitsäuredianhydrid aus dieses enthaltenden Reaktionsgasen in form freifließender Kristalle, wobei die in bekannter Weise vorgekühlten Reaktionsgase zur weiteren an sich bekannten Abkühlung auf Temperaturen oberhalb des Taupunktes der Nebenprodukte gebracht werden, dadurch gekennzeichnet, daß die Reaktionsgase mit niedrigen Strömungsgeschwindigkeiten von maximal 3 m/sec., insbesondere maximal 1 m/sec., parallel zu glattflächigen, thermostatisierten Kühlflächen geführt werden.

2. Verfahren nach Anspruch 1, dadurch gekennzeichnet, daß die Reaktionsgase senkrecht von oben nach unten durch die Rohre eines thermostatisierten Rohrbündels geführt werden.

3. Verfahren nach Anspruch 1, dadurch gekennzeichnet, daß die Reaktionsgase parallel zu den Wärmeaustauschflächen eines Plattenkühlers geführt werden.

### Revendications

1. Procédé pour la sublimation fractionnée du dianhydride de l'acide pyromellitique ultra pur, à partir de gaz réactionnels le contenant, sous forme de cristaux fluides, les gaz réactionnels préalablement refroidis de manière connue étant portés ensuite, par refroidissement connu en soi, à des températures situées au-dessus du point de rosée des produits secondaires, procédé caractérisé en ce que les gaz réactionnels sont conduits avec des faibles vitesses d'ecoulement de maximum 3 m/sec, de préférence maximum 1 m/sec, en direction parallèle aux surfaces de refroidissement lisses et thermostatées.

2. Procédé selon la revendication 1, caractérisé en ce que les gaz réactionnels sont conduits en direction verticale, de haut en bas, à travers les tubes d'un faisceau de tubes thermostaté.

3. Procédé selon la revendication 1, caractéisé en ce que les gaz réactionnels son conduits en direction parallèle aux surfaces d'echange thermique d'un refroidisseur à plaques.

## Claims

1. A process for the fractional desublimation of very pure pyromellitic acid dianhydride from reaction gases containing said substance, in the form of free-flowing crystals, in which case the reaction gases that are pre-cooled in a known manner, for the purpose of a further cooling, known in itself, are brought to temperatures above the thaw point of the by-products, wherein the reaction gases at low flow velocities of maximally 3 m/sec., in particular, maximally 1 m/sec., are directed parallel to the smooth, thermostatized cooling surfaces.

2. Process according to Claim 1, wherein said reaction gases are led vertically downward through the pipes of a thermostatized pipe assembly.

3. Process according to Claim 1, wherein said reaction gases are directed parallel to the heat exchange surfaces of a plate cooler.